# FASCICULE DE BREVET EUROPEEN

(11) **EP 3 354 327 B1**
(45) Date de publication et mention de la délivrance du brevet: **22.07.2020**
(21) Numéro de dépôt: 18151043.9
(22) Date de dépôt: 17.04.2012
(51) Int. Cl.: B01D 53/14, C07C 7/10, C07C 7/11

(54) **RÉCUPÉRATION DE MONOMÈRES**
RÜCKGEWINNUNG VON MONOMEREN
MONOMER RECOVERY

(30) Priorité: 22.04.2011 FR 1153493
(43) Date de publication de la demande: 01.08.2018
(62) Demande divisionnaire de: 12713770.1
(73) Titulaire: Compagnie Générale des Etablissements Michelin, 63000 Clermont-Ferrand (FR)
(72) Inventeur: DORATO, Margarita, 63040 Clermont-Ferrand Cedex 09 (FR); KIENER, Pierre, 63040 Clermont-Ferrand Cedex 09 (FR)
(74) Mandataire: Le Cam, Véronique Marie Christine

(56) Documents cités:
- WO-A1-89/03009
- GB-A- 570 870
- GB-A- 2 063 291
- US-A- 2 409 250

## Description

La présente invention se rapporte à un procédé de récupération de monomères diéniques ou vinyle aromatiques à partir d'un flux de gaz, ce procédé de récupération comprenant une étape d'extraction par mise en contact du flux de gaz avec un solvant d'extraction organique afin d'absorber le ou les monomères, puis une étape de récupération dans laquelle le ou les monomères sont séparés du solvant d'extraction, ce dernier étant ensuite recyclé pour alimenter l'étape d'extraction.

Dans le domaine de l'extraction et/ou de la récupération des hydrocarbures contenus dans un flux de gaz dilué, il existe principalement quatre types de procédé d'extraction : les procédés de séparation cryogéniques, les procédés de séparation par membranes sélectives, les procédés d'adsorption/désorption des monomères sur supports sélectifs et les procédés d'absorption des monomères en solvants sélectifs.

Les procédés de séparation cryogéniques sont bien connus de l'homme de l'art, mais ces procédés sont coûteux et nécessitent des appareils permettant d'opérer à très basse température et souvent à haute pression.

L'extraction des monomères d'un flux de gaz par des membranes sélectives a été proposée comme alternative à la séparation cryogénique. Par exemple, le brevet US 5,769,927 décrit un procédé de récupération de monomères du gaz de purge en trois étapes : condensation, flash évaporation et séparation par membrane. Mais les deux premières étapes nécessitent cependant un traitement cryogénique à des températures très basses (-60°C) et à des pressions élevées (35 bar).

L'extraction des monomères d'un flux de gaz par adsorption/désorption a également été étudiée en tant qu'alternative à la séparation cryogénique. Par exemple, le brevet EP 1 302 478 décrit la récupération de monomères par adsorption et désorption sur gel de silice ou alumine. La récupération du monomère est effectuée par un procédé PSA (Pressure Swing Adsorption). Mais ce type de procédé nécessite néanmoins généralement de faibles pressions, de l'ordre de 0,1 bar pour la désorption du monomère.

De plus, l'utilisation des membranes sélectives ou des supports adsorbants pourrait entraîner l'homopolymérisation de certains monomères.

La séparation des hydrocarbures légers d'un flux de gaz en utilisant des solvants d'extraction est également connue, notamment pour récupérer du méthane, de l'éthane, de l'éthylène, du propylène.

Ainsi, il existe de nombreuses installations industrielles permettant le fractionnement de charges gazeuses en un gaz résiduaire.

On connaît ainsi du document US 2,409,250 un procédé pour séparer le butadiène d'un mélange gazeux contenant du butadiène et des hydrocarbures. Ce procédé comprend une étape de purification du mélange avec un solvant pour obtenir une solution contenant le butadiène et un gaz substantiellement exempt de butadiène, et à distiller la solution pour séparer le butadiène.

On connaît également du document WO 89/03009 un procédé pour séparer des composés d'un flux de gaz hydrocarbures, notamment des hydrocarbures insaturés par mise en contact à contre-courant, dans une colonne de séparation, du flux de gaz hydrocarbures avec un solvant, et une étape de récupération du solvant appauvri à partir du solvant enrichi issu du bas de la colonne de séparation. Un autre exemple est donné dans GB570870A.

Mais cependant il existe encore un besoin de récupérer des monomères diéniques ou d'autres monomères avec lesquels ces monomères diéniques sont parfois co-polymérisés pour préparer des élastomères, qui seraient contenus dans des flux de gaz, de façon simple, économique, flexible et polyvalente, avec de plus une pureté telle qu'ils soient directement utilisables en polymérisation, en particulier pour la fabrication de pneumatiques.

L'homme de l'art sait que la pureté d'un monomère est un facteur déterminant pour envisager son utilisation en polymérisation. Dans un flux de gaz, des impuretés plus ou moins néfastes pour la polymérisation peuvent être présentes.

Trois grandes familles d'impuretés plus ou moins néfastes pour les polymérisations décrites précédemment peuvent être présents dans les flux de gaz contenant lesdits monomères : (I) les gaz tels que le dioxyde de carbone, le monoxyde de carbone et l'oxygène, (II) les dérivés organiques possédant une ou plusieurs doubles liaisons tels que des oligomères du monomère cible et (III) les dérivés portant un hétéroatome comme l'oxygène, le soufre ou l'azote, tels que l'eau, les aldéhydes, les acétals, les alcools, les cétones, les éthers, les amines et les thiols.

Ces impuretés peuvent nuire au rendement de la polymérisation, générer des réactions parasites altérant les propriétés du produit final, diminuer l'activité du catalyseur ce qui nécessiterait une quantité plus importante ou même inhiber toute réaction.

A titre d'exemple on peut citer l'effet de certaines impuretés dans la polymérisation du 1,4 cis-polyisoprène préparé à base d'un métal de transition: 10 ppm de dioxyde de carbone dans l'isoprène suffisent à inhiber la polymérisation. Des impuretés protiques telles que l'eau ou l'éthanol doivent être compensées par un excès du catalyseur pour éviter une diminution de l'activité. Ainsi il a été observé dans des conditions identiques de polymérisation que la présence de 15 ppm d'eau dans de l'isoprène abaissait la conversion de 10 points.

C'est pour cela qu'il est important de réduire au minimum la teneur de ces impuretés dans le monomère lors de la phase de récupération, afin de simplifier voire de supprimer des étapes ultérieures de purification.

La demanderesse a mis au point un procédé de récupération qui s'applique aux monomères diéniques ainsi qu'à certains monomères avec lesquels ces monomères diéniques sont parfois polymérisés pour préparer des élastomères notamment de grade pneumatique, tels que les monomères vinyle aromatiques ou l'isobutène, et qui apporte une solution au problème exposé précédemment.

Ainsi, l'invention a pour objet un procédé de récupération d'un ou plusieurs monomères à partir d'un flux de gaz, comprenant les étapes suivantes :
au sein d'une même première colonne d'extraction Cl,
a) une étape d'extraction par mise en contact dans une colonne d'extraction C1 du flux de gaz avec un solvant d'extraction organique, dans laquelle ledit solvant d'extraction absorbe le ou lesdits monomères, et
b) une étape de stripage ou de désorption avec un gaz inerte dans la colonne d'extraction C1 par alimentation en pied de la colonne Cl, et en dessous de l'alimentation du flux de gaz chargé en monomères, par un flux de gaz inerte, de préférence du diazote ou un flux de gaz enrichi en diazote,
   un flux liquide comprenant le solvant d'extraction et du ou des monomères étant récupéré en pied de la colonne Cl, un flux de gaz sortant étant récupéré en tête de la colonne Cl, puis
   au sein d'une deuxième colonne de récupération C2,
c) une étape de récupération du ou desdits monomères, dans laquelle le ou lesdits monomères sont séparés du solvant d'extraction par distillation dans une colonne de récupération C2 alimentée par le flux liquide récupéré en pied de la colonne Cl, un flux comprenant du ou des monomères concentrés étant récupéré en tête de la colonne C2, et un flux liquide comprenant le solvant d'extraction étant récupéré en pied de la colonne C2 puis recyclé en tête de la colonne Cl,
   le ou les monomères étant choisis parmi les diènes, les vinyles aromatiques et l'isobutène.

En particulier, le ou les monomères récupérés par le procédé selon l'invention peuvent être choisis parmi l'isoprène, le butadiène, l'isobutène et le styrène.

Le solvant d'extraction est généralement choisi parmi les hydrocarbures aliphatiques, cycloaliphatiques et aromatiques, ayant de 5 à 20 atomes de carbone, préférentiellement ayant de 6 à 15 atomes de carbone et plus préférentiellement ayant de 6 à 10 atomes de carbone. On utilisera de préférence un solvant qui n'est pas une impureté pour la polymérisation des élastomères utilisés pour la fabrication de pneumatiques. A titre de solvant d'extraction particulièrement préféré, on peut citer le n-hexane, des coupes d'isomères d'hexane, le cyclohexane, le n-heptane, des coupes d'isomères d'heptane, le méthylcyclohexane et le toluène.

Le procédé selon l'invention peut également comprendre une étape de condensation du flux de gaz sortant récupéré en tête de la colonne C1 suivie d'une étape de séparation, pour récupérer d'une part une purge de gaz et d'autre part une solution d'hydrocarbures condensés comprenant du solvant d'extraction et du ou des monomères, ladite solution étant recyclée en tête de la colonne C1.

Le flux liquide comprenant le solvant d'extraction et du ou des monomères récupéré en pied de la colonne C1 peut être chauffé dans un échangeur de chaleur E3 par le flux liquide comprenant le solvant d'extraction récupéré en pied de la colonne C2.

Le procédé selon l'invention peut également comprendre une étape de condensation du flux comprenant du ou des monomères concentrés récupéré en tête de la colonne C2, suivi d'une étape de séparation, pour récupérer d'une part des composés incondensables et d'autre part un flux de monomère(s), une partie du flux de monomère(s) étant réinjecté en tête de la colonne C2.

Le rapport entre le débit de flux de monomère(s) réinjecté en tête de la colonne C2 et le débit de flux de monomère(s) non réinjecté en tête de la colonne C2 est appelé taux de reflux. Le taux de reflux est généralement compris entre 0,001 et 50.

Les composés incondensables peuvent être recyclés en pied de la colonne C1.

Une partie du flux liquide comprenant le solvant d'extraction récupéré en pied de la colonne C2 peut être vaporisé par un échangeur E5 et réinjecté en pied de la colonne C2.

Selon un mode de réalisation particulier, le flux liquide comprenant le solvant d'extraction et du ou des monomère(s) récupéré en pied de la colonne C1 subi une étape de séparation, pour récupérer dudit solvant une partie des composés incondensables, lesdits composés incondensables étant réintroduits en pied de la colonne C1.

Selon un autre mode de réalisation particulier, une partie du flux liquide comprenant le solvant d'extraction et du ou des monomères récupéré en pied de la colonne C1 est vaporisé dans un échangeur de chaleur E6 et est réintroduit en pied de la colonne C1.

Les monomères obtenus par le procédé selon l'invention peuvent être avantageusement utilisés pour la synthèse de polymères eux-mêmes utilisables pour la fabrication de pneumatiques.

D'autres avantages et caractéristiques apparaîtront à l'examen de la description détaillée de modes de réalisation de l'invention, nullement limitatifs, et des dessins annexés sur lesquels :
- la figure 1 illustre de façon schématique le procédé général selon l'invention ;
- la figure 2 illustre de façon schématique un mode de réalisation du procédé selon l'invention ;
- la figure 3 illustre de façon schématique un autre mode de réalisation du procédé selon l'invention, dans lequel en particulier les composés incondensables récupérés après la distillation sont recyclés dans la colonne d'extraction C1 ;
- la figure 4 illustre de façon schématique un autre mode de réalisation du procédé selon l'invention ;
- la figure 5 illustre de façon schématique un autre mode de réalisation du procédé selon l'invention.

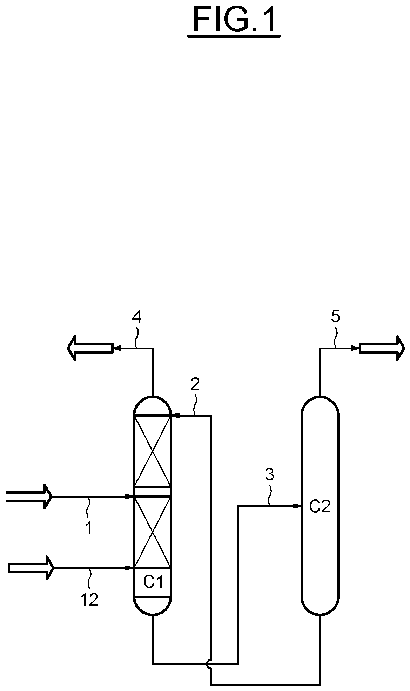

Sur la figure 1, on a représenté de manière schématique le procédé selon l'invention.

Le flux de gaz 1 à partir duquel sont récupérés les monomères peut provenir, de manière non limitative, d'une purge suite à une étape de polymérisation, par exemple, une purge de gaz d'une boucle de refroidissement de la réaction de polymérisation par évaporation, d'une étape de concentration de solution de polymère, par exemple, tout étape d'élimination des monomères non réagi et du solvant de réaction d'une solution de polymère par exemple avec un agent d'entraînement tel que l'azote, d'une purge suite à une étape d'épuration des monomères, par exemple d'une épuration par entraînement avec un agent de stripage ou la récupération de la phase gaz d'une colonne de distillation azéotropique ou d'un bioréacteur, tel qu'un fermenteur ou un photobioréacteur.

Le flux de gaz 1 comprend généralement à titre de composants principaux, mais nullement limitatifs, du diazote, du monoxyde de carbone, du dioxyde de carbone, du dioxygène, de l'argon, du dihydrogène, de l'eau et des hydrocarbures dont le ou les monomères à récupérer. Le flux de gaz 1 peut comprendre à titre de composants minoritaires, des dérivés portant un hétéroatome comme l'oxygène, le soufre ou l'azote, tels que l'eau, des aldéhydes, des acétals, des alcools, des éthers, des cétones, des amines ou des thiols. Il peut aussi comprendre des autres hydrocarbures dont les solvants de polymérisation.

Le flux de gaz 1 alimente le milieu de la colonne d'extraction C1 dans laquelle il est mis en contact avec le solvant d'extraction 2 alimenté en tête de colonne. Le solvant absorbe le ou lesdits monomères. Un flux de gaz inerte 12, tel que du diazote ou un flux de gaz enrichi en diazote, est alimenté en pied de la colonne C1. Ce gaz inerte facilite la désorption des composés légers et potentiellement néfaste pour la polymérisation, tels que le dioxygène, le monoxyde de carbone, le dioxyde de carbone et l'eau, entre autres. Ainsi, la colonne C1 présente une zone d'absorption au-dessus du niveau d'alimentation du flux de gaz 1, dans laquelle les composants lourds du flux de gaz 1 sont transférés de la phase gazeuse vers la phase liquide afin d'enrichir cette phase en monomère(s), et une zone de désorption au-dessous du niveau d'alimentation du flux de gaz 1, dans laquelle certains composants de la phase liquide sont transférés à la phase gazeuse afin d'appauvrir ladite phase liquide des composants légers, tels que le dioxyde de carbone, le monoxyde de carbone, le dioxygène et l'eau, entre autres.

On récupère alors en pied de la colonne C1 un flux liquide 3 comprenant principalement le solvant d'extraction et du ou des monomères, et en tête de la colonne C1 un flux de gaz sortant 4. Le flux de gaz sortant 4 a une teneur très faible en monomère. Il comprend essentiellement des gaz présents dans le flux 1, du solvant et une quantité réduite du ou des monomères, ainsi que du gaz inerte provenant du stripage. L'efficacité de récupération de la colonne C1 peut atteindre 99,99%, c'est-à-dire que le flux liquide 3 récupéré en pied de la colonne C1 peut comprendre jusqu'à 99,99% en poids du ou des monomères initialement présents dans le flux de gaz 1.

Le flux liquide 3 comprenant le solvant d'extraction et du ou des monomères est alors conduit vers la colonne C2.

Le ou les monomères sont séparés du solvant d'extraction par distillation dans la colonne de récupération C2. Le flux de monomère(s) concentré 5 est récupéré en tête de la colonne C2. La teneur en monomère(s) dans le flux de monomère(s) concentré 5 peut atteindre 99,99% en poids. Le flux liquide 2 comprenant le solvant d'extraction est récupéré en pied de la colonne C2 et est recyclé en tête de la colonne C1.

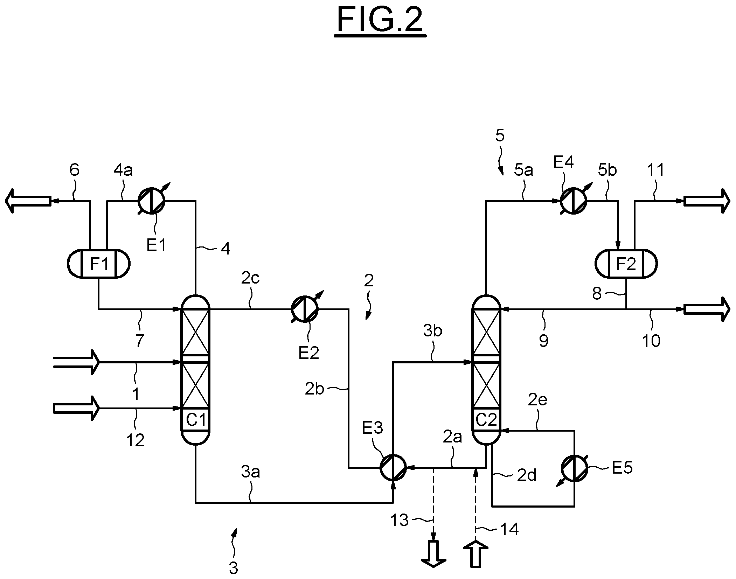

Sur la figure 2, on a représenté de façon schématique un mode de réalisation économique et flexible du procédé selon l'invention. Les références de la figure 1 ont été reportées sur cette figure 2. Le flux liquide 3a est chauffé dans un échangeur de chaleur E3 par le flux liquide 2a, le flux de liquide 3b, chauffé, qui sort de l'échangeur E3 est dirigé vers la colonne C2. De la même façon, le flux liquide 2a récupéré en pied de la colonne C2 est dirigé vers l'échangeur E3 et le flux liquide 2b qui sort refroidi de l'échangeur E3 est dirigé vers un échangeur E2, et le flux liquide 2c qui sort de l'échangeur E2, est conditionné en température par ce dernier et est introduit en tête de la colonne C1.

Le flux de gaz sortant 4 est récupéré en tête de la colonne C1. Il comprend essentiellement des gaz présents dans le flux 1, du solvant et une quantité réduite de monomère(s), ainsi que du gaz inerte provenant du stripage. Il subi une étape de condensation dans un condenseur E1, afin de récupérer d'éventuels hydrocarbures entraînés par la phase gaz. Ce flux de gaz 4a est séparé alors dans le bidon F1, afin de récupérer d'une part une purge de gaz 6 comprenant la majorité des gaz présents dans le flux 1, une quantité réduite du solvant et des éventuelles traces de monomère(s) et d'autre part un flux liquide 7 d'hydrocarbures condensés comprenant du solvant d'extraction et une quantité réduite de monomère(s). Le flux liquide 7 est recyclé en tête de la colonne C1.

De la même façon, le flux de monomère(s) concentré 5a récupéré en tête de la colonne C2 est condensé totalement ou partiellement dans un condenseur E4, pour donner un flux de gaz 5b qui est séparé dans le bidon F2, pour récupérer d'une part des composés incondensables 11 provenant des gaz solubilisés dans le flux liquide 3a et d'autre part un flux de monomère(s) 8. Le flux de monomère(s) contient éventuellement, outre le ou les monomères, une faible quantité de diazote, une faible quantité de solvant d'extraction et éventuellement des traces de gaz tels que du dioxyde de carbone, du dioxygène entre autres. Une partie 9 du flux de monomère(s) est réinjecté assurant le reflux en tête de la colonne C2, l'autre partie 10 étant le distillat. Le taux de reflux, rapport entre le débit du flux 9 et le débit du flux 10, est défini pour atteindre une pureté de monomère(s) du flux 10 pouvant atteindre 99,99%. Le taux de reflux est généralement compris entre 0,001 et 50. Cette valeur peut varier selon le dimensionnement de la colonne C2, comme cela est connu de l'homme de l'art.

Une partie 2d du flux liquide comprenant le solvant d'extraction récupéré en pied de la colonne C2 est vaporisé par un échangeur E5 et est réinjecté en pied de la colonne C2, ce qui assure le rebouillage dans la colonne. Le taux de rebouillage, rapport entre le débit de la partie vaporisée du flux 2e et le débit du flux 2a, est fixé en fonction de la concentration de monomère(s) souhaitée dans le flux 2a en pied de colonne C2. Le taux de rebouillage est généralement compris entre 0,01 et 30. Cette valeur peut varier selon le dimensionnement de la colonne C2, comme cela est connu de l'homme de l'art.

La colonne C2 présente classiquement une zone de concentration au-dessus du niveau d'alimentation du flux liquide 3b, dans laquelle la vapeur générée dans le rebouilleur E3 s'enrichit en monomère, et une zone d'épuisement au-dessous du niveau d'alimentation du flux liquide 3b, dans laquelle le liquide du flux 9 provenant du reflux s'appauvrit en monomère(s).

Il est possible d'introduire du solvant d'extraction frais 14 au niveau de la ligne de flux liquide 2a afin de pouvoir faire un appoint de solvant selon les besoins.

Il est également possible de prévoir une purge de composants lourds 13 au niveau de la ligne de flux liquide 2a. Ce flux de purge peut être obtenu par tout dispositif adapté, y compris ceux permettant la purification du solvant d'extraction qui peut alors être injecté à nouveau au point 14.

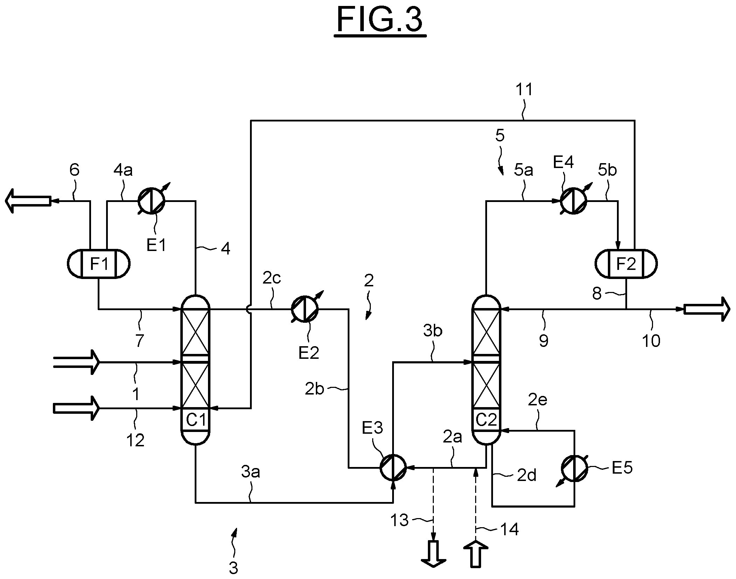

Sur la figure 3, on a représenté de façon schématique un mode de réalisation du procédé selon l'invention. Les références de la figure 2 ont été reportées sur cette figure 3.

Dans ce mode de réalisation, les composés incondensables 11 sont recyclés au pied de la colonne C1.

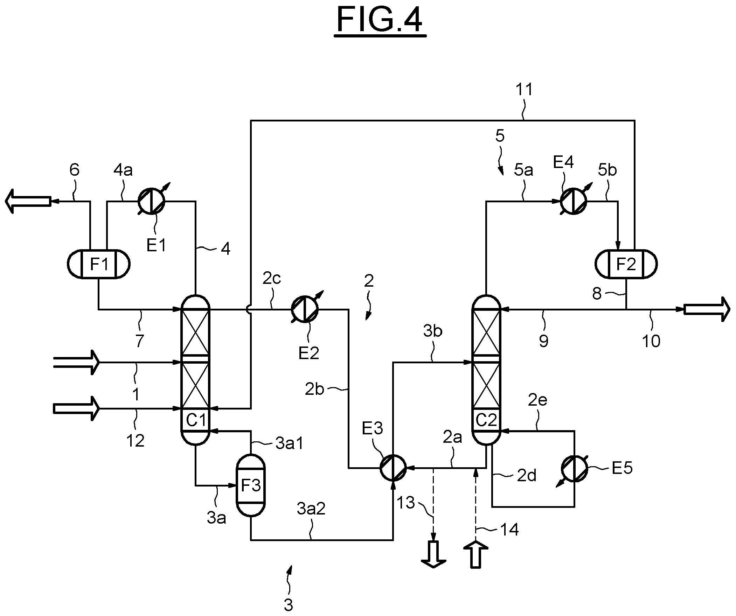

Sur la figure 4, on a représenté de façon schématique un mode de réalisation du procédé selon l'invention. Les références de la figure 3 ont été reportées sur cette figure 4.

Le flux liquide 3a comprenant le solvant d'extraction et du ou des monomères récupérés en pied de colonne C1 est séparé dans un bidon F3, pour d'une part obtenir une partie des composés incondensables 3a1 qui sont réintroduits en pied de la colonne Cl, et d'autre part récupérer un flux liquide 3a2 comprenant le solvant d'extraction et du ou des monomères qui est conduit vers l'échangeur E3.

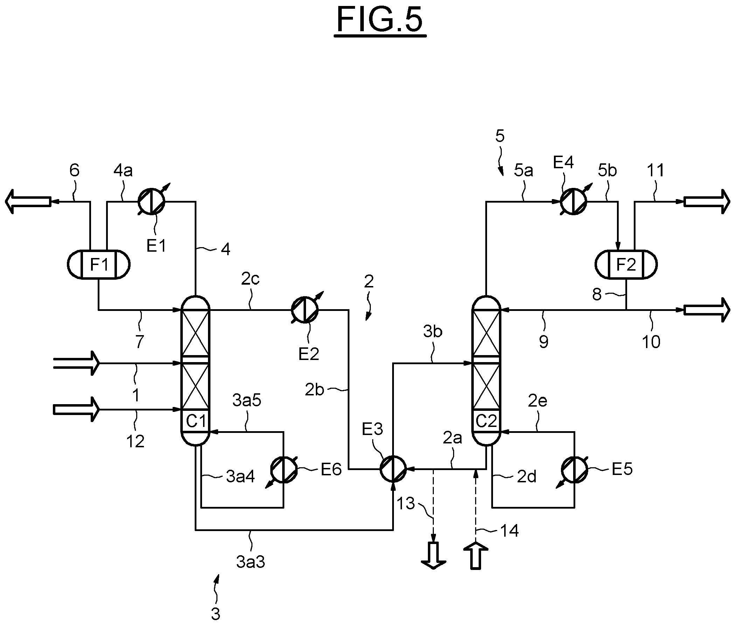

Sur la figure 5, on a représenté de façon schématique un mode de réalisation du procédé selon l'invention. Les références de la figure 2 ont été reportées sur cette figure 5.

Une partie 3a4 du flux liquide comprenant le solvant d'extraction et du ou des monomères récupéré en pied de la colonne C1 est vaporisé par un échangeur E6 pour former un flux 3a5 qui est réinjecté en pied de la colonne Cl, ce qui assure un rebouillage éventuel dans la colonne. Le taux de rebouillage, rapport entre le débit de la partie vaporisée du flux 3a5 et le débit du flux 3a4, est fixé en fonction de la concentration de composants légers autre que le ou les monomères souhaitée dans le flux 3a3 en pied de colonne C1. Le taux de rebouillage est généralement compris entre 0,01 et 30. Cette valeur peut varier selon le dimensionnement de la colonne Cl, comme cela est connu de l'homme de l'art. Le flux 3a3 en pied de colonne C1 est envoyé vers l'échangeur E3.

Dans tous les modes de réalisation de l'invention, la qualité du ou des monomères obtenus dépend des conditions mises en œuvre dans les colonnes C1 et C2 : températures, pressions, choix du solvant d'extraction en fonction de la nature du flux de gaz, ratio entre débit de flux de gaz et le flux de solvant, taux de reflux, taux de rebouillage, nombre de plateaux. Les choix de ces paramètres donnent un grand degré d'efficacité et de flexibilité au procédé selon l'invention. Les températures peuvent être comprise entre -30°C et 200°C, les pressions peuvent être comprise entre 0,5 et 10 bar (1 bar = 100kPa). Le nombre des plateaux peut être compris entre 2 et 50.

L'invention est illustrée par les exemples suivants.

### Exemple 1

Un procédé de récupération d'isoprène selon l'invention est étudié par modélisation sur le logiciel ASPEN Plus ™. La mise en œuvre du procédé se fait selon le mode de réalisation de la figure 4. Le solvant d'extraction utilisé est le cyclohexane.

Les conditions de fonctionnement de la colonne d'extraction C1 sont données dans le tableau 1.

**Tableau 1**

| | |
|---|---|
| Nombre de plateaux théoriques | 20 |
| Température (°C) | 10 |
| Pression (bar) | 2 |
| Rapport débit massique cyclohexane/débit massique flux gaz entrée | 3 |

Les conditions de fonctionnement de la colonne de récupération C2 sont données dans le tableau 2.

**Tableau 2**

| | |
|---|---|
| Nombre de plateaux théoriques | 20 |
| Plateau alimentation | 17 |
| Pression (bar) | 2,5 |
| Taux de reflux | 1,36 |
| Taux de rebouillage | 3 |

Les compositions des flux entrant et sortant sont données dans le tableau 3.

**Tableau 3**

| | Flux entrants | | | Flux sortant | |
|---|---|---|---|---|---|
| Ligne-référence | 1 | 12 | 14 | 6 | 10 |
| Description | Flux gaz entrée | Diazote | Appoint solvant | Purge gaz | Isoprène purifié |

| Fraction massique | | | | | |
|---|---|---|---|---|---|
| Composant | | | | | |
| N₂ | 0,504 | 1 | | 0,641 | 0,002 |
| O₂ | 0,088 | | | 0,07 | 0 |
| CO₂ | 0,286 | | | 0,228 | 0 |
| Ar | 0,009 | | | 0,007 | 0 |
| Isoprène | 0,112 | | | 0,001 | 0,998 |
| Cyclohexane | | | 1 | 0,051 | 0 |
| Eau | 0,001 | | | 0,001 | 0 |

| Rapport débits | | | | | |
|---|---|---|---|---|---|
| w/wI0 | 8,9 | 2,7 | 0,6 | 11,2 | 1,0 |
| w/wFG0 | 1,0 | 0,3 | 0,1 | 1,3 | 0,1 |

| | | | | | |
|---|---|---|---|---|---|
| w/wI0 : débit massique du flux/débit massique d'isoprène dans le flux gaz entrée w/wFG₀ : débit massique du flux/débit massique du flux gaz entrée | | | | | |

### Exemple 2

Un procédé de récupération d'isoprène selon l'invention est étudié par modélisation sur le logiciel ASPEN Plus ™. La mise en œuvre du procédé se fait selon le mode de réalisation de la figure 4. Le solvant d'extraction utilisé est le cyclohexane.

Les conditions de fonctionnement de la colonne d'extraction C1 sont données dans le tableau 4.

**Tableau 4**

| | |
|---|---|
| Nombre de plateaux théoriques | 20 |
| Température (°C) | 10 |
| Pression (bar) | 2 |
| Rapport débit massique cyclohexane/débit massique flux gaz entrée | 4 |

Les conditions de fonctionnement de la colonne de récupération C2 sont données dans le tableau 5.

**Tableau 5**

| | |
|---|---|
| Nombre de plateaux théoriques | 20 |
| Plateau alimentation | 17 |
| Pression (bar) | 2,5 |
| Taux de reflux | 3,1 |
| Taux de rebouillage | 0,08 |

Les compositions des flux entrant et sortant sont données dans le tableau 6.

**Tableau 6**

| | Flux entrants | | | Flux sortant | |
|---|---|---|---|---|---|
| Ligne-référence | 1 | 12 | 14 | 6 | 10 |
| Description | Flux gaz entrée | Diazote | Appoint solvant | Purge gaz | Isoprène purifié |

| Fraction massique | | | | | |
|---|---|---|---|---|---|
| Composant | | | | | |
| N₂ | 0,504 | 1 | | 0,642 | 0,002 |
| O₂ | 0,088 | | | 0,07 | 0 |
| CO₂ | 0,286 | | | 0,228 | 0 |
| Ar | 0,009 | | | 0,007 | 0 |
| Isoprène | 0,112 | | | 0 | 0,981 |
| Cyclohexane | | | 1 | 0,051 | 0,017 |
| Eau | 0,001 | | | 0,001 | 0 |

| Rapport débits | | | | | |
|---|---|---|---|---|---|
| w/wI0 | 8,9 | 2,7 | 0,6 | 11,2 | 1,0 |
| w/wFG0 | 1,0 | 0,3 | 0,1 | 1,3 | 0,1 |

| | | | | | |
|---|---|---|---|---|---|
| w/wI0 : débit massique du flux/débit massique d'isoprène dans le flux gaz entrée w/wFG₀ : débit massique du flux/débit massique du flux gaz entrée | | | | | |

## Revendications

1. Procédé de récupération d'un ou plusieurs monomères à partir d'un flux de gaz (1), comprenant les étapes suivantes :
au sein d'une même première colonne d'extraction Cl,
a) une étape d'extraction par mise en contact dans une colonne d'extraction (C1) du flux de gaz (1) avec un solvant d'extraction organique (2), dans laquelle ledit solvant d'extraction (2) absorbe le ou lesdits monomères, et
b) une étape de stripage ou de désorption avec un gaz inerte dans la colonne d'extraction (C1) par alimentation en pied de la colonne (C1), et en dessous de l'alimentation du flux de gaz (1) chargé en monomères, par un flux de gaz inerte, de préférence du diazote ou un flux de gaz enrichi en diazote (12),
un flux liquide (3) comprenant le solvant d'extraction et du ou des monomères étant récupéré en pied de la colonne (C1), un flux de gaz sortant (4) étant récupéré en tête de la colonne (C1), puis
au sein d'une deuxième colonne de récupération C2,
c) une étape de récupération du ou desdits monomères, dans laquelle le ou lesdits monomères sont séparés du solvant d'extraction par distillation dans une colonne de récupération (C2) alimentée par le flux liquide (3) récupéré en pied de la colonne (C1), un flux comprenant du ou des monomère(s) concentrés (5) étant récupéré en tête de la colonne (C2), et un flux liquide (2) comprenant le solvant d'extraction étant récupéré en pied de la colonne (C2) puis recyclé en tête de la colonne (C1),
le ou les monomères étant choisis parmi les diènes, les vinyles aromatiques et l'isobutène.

2. Procédé selon la revendication 1 **caractérisé en ce que** le ou les monomères sont choisis parmi l'isoprène, le butadiène, l'isobutène et le styrène.

3. Procédé selon la revendication 1 ou 2 **caractérisé en ce que** le solvant d'extraction est choisi parmi les hydrocarbures aliphatiques, cycloaliphatiques et aromatiques, ayant de 5 à 20 atomes de carbone.

4. Procédé selon la revendication 3 **caractérisé en ce que** le solvant d'extraction est choisi parmi le n-hexane, des coupes d'isomères d'hexane, le cyclohexane, le n-heptane, des coupes d'isomères d'heptane, le méthylcyclohexane et le toluène.

5. Procédé selon l'une quelconque des revendications précédentes, **caractérisé en ce qu'**il comprend une étape de condensation du flux de gaz sortant (4) récupéré en tête de la colonne (C1) suivie d'une étape de séparation, pour récupérer d'une part une purge de gaz (6) et d'autre part une solution (7) d'hydrocarbures condensés comprenant du solvant d'extraction et du ou des monomères, ladite solution (7) étant recyclée en tête de la colonne (C1).

6. Procédé selon l'une quelconque des revendications précédentes, **caractérisé en ce que** le flux liquide (3) comprenant le solvant d'extraction et du ou des monomères récupéré en pied de la colonne (C1) est chauffé dans un échangeur de chaleur E3 par le flux liquide (2) comprenant le solvant d'extraction récupéré en pied de la colonne (C2).

7. Procédé selon l'une quelconque des revendications précédentes, **caractérisé en ce qu'**il comprend une étape de condensation du flux comprenant du ou des monomère(s) concentrés (5) récupéré en tête de la colonne (C2), suivi d'une étape de séparation, pour récupérer d'une part des composés incondensables (11) et d'autre part un flux de monomère(s) (8), une partie (9) du flux de monomère(s) étant réinjecté en tête de la colonne (C2).

8. Procédé selon la revendication 7 **caractérisé en ce que** les composés incondensables (11) sont recyclés en pied de la colonne (C1).

9. Procédé selon l'une quelconque des revendications précédentes, **caractérisé en ce que** le flux liquide comprenant le solvant d'extraction et du ou des monomères récupéré en pied de la colonne (C1) subi une étape de séparation, pour séparer dudit solvant une partie des composés incondensables, lesdits composés incondensables étant réintroduits en pied de la colonne (C1).

10. Procédé selon l'une quelconque des revendications 1 à 8 **caractérisé en ce que** une partie du flux liquide comprenant le solvant d'extraction et du ou des monomères récupéré en pied de la colonne (C1) est vaporisé dans un échangeur de chaleur E6 et est réintroduit en pied de la colonne (C1).

## Patentansprüche

1. Verfahren zur Gewinnung eines oder mehrerer Monomere aus einem Gasstrom (1), das die folgenden Schritte umfasst:
in derselben ersten Extraktionskolonne C1
a) einen Schritt der Extraktion durch Inkontaktbringen des Gasstroms (1) in einer Extraktionskolonne (C1) mit einem organischen Extraktionslösungsmittel (2), wobei das Extraktionslösungsmittel (2) das bzw. die Monomere absorbiert, und
b) einen Schritt des Strippens oder Desorbierens mit einem Inertgas in der Extraktionskolonne (C1) durch Einspeisen eines Stroms von Inertgas, vorzugsweise Distickstoff oder einem mit Distickstoff angereicherten Gasstrom (12), am Sumpf der Kolonne (C1) und unterhalb der Einspeisung des mit Monomeren beladenen Gasstroms (1),
wobei am Sumpf der Kolonne (C1) ein flüssiger Strom (3), der das Extraktionslösungsmittel und Monomer bzw. Monomere umfasst, gewonnen wird und am Kopf der Kolonne (C1) ein Austrittsgasstrom (4) gewonnen wird, dann in einer zweiten Rückgewinnungskolonne C2
c) einen Schritt der Rückgewinnung des bzw. der Monomere, bei dem das bzw. die Monomere durch Destillation in einer mit dem am Sumpf der Kolonne (C1) gewonnenen flüssigen Strom (3) gespeisten Rückgewinnungskolonne (C2) von dem Extraktionslösungsmittel getrennt werden, wobei am Kopf der Kolonne (C2) ein konzentriertes Monomer bzw. konzentrierte Monomere umfassender Strom (5) gewonnen wird und am Sumpf der Kolonne (C2) ein das Extraktionslösungsmittel umfassender flüssiger Strom (2) gewonnen und dann zum Kopf der Kolonne (C1) zurückgeführt wird,
wobei das bzw. die Monomere aus Dienen, Vinylaromaten und Isobuten ausgewählt werden.

2. Verfahren nach Anspruch 1, **dadurch gekennzeichnet, dass** das bzw. die Monomere aus Isopren, Butadien, Isobuten und Styrol ausgewählt werden.

3. Verfahren nach Anspruch 1 oder 2, **dadurch gekennzeichnet, dass** das Extraktionslösungsmittel aus aliphatischen, cycloaliphatischen und aromatischen Kohlenwasserstoffen mit 5 bis 20 Kohlenstoffatomen ausgewählt wird.

4. Verfahren nach Anspruch 3, **dadurch gekennzeichnet, dass** das Extraktionslösungsmittel aus n-Hexan, Hexanisomerenschnitten, Cyclohexan, n-Heptan, Heptanisomerenschnitten, Methylcyclohexan und Toluol ausgewählt wird.

5. Verfahren nach einem der vorhergehenden Ansprüche, **dadurch gekennzeichnet, dass** es einen Schritt der Kondensation des am Kopf der Kolonne (C1) gewonnenen Austrittsgasstroms (4) gefolgt von einem Trennungsschritt zur Gewinnung eines Teils einer Gasausschleusung (6) einerseits und einer Lösung (7) von kondensierten Kohlenwasserstoffen, die Extraktionslösungsmittel und Monomer bzw. Monomere umfasst, andererseits umfasst, wobei die Lösung (7) zum Kopf der Kolonne (C1) zurückgeführt wird.

6. Verfahren nach einem der vorhergehenden Ansprüche, **dadurch gekennzeichnet, dass** der am Sumpf der Kolonne (C1) gewonnene flüssige Strom (3), der das Extraktionslösungsmittel und Monomer bzw. Monomere umfasst, in einem Wärmetauscher E3 durch den am Sumpf der Kolonne (C2) gewonnenen flüssigen Strom (2), der das Extraktionslösungsmittel umfasst, erwärmt wird.

7. Verfahren nach einem der vorhergehenden Ansprüche, **dadurch gekennzeichnet, dass** es einen Schritt der Kondensation des am Kopf der Kolonne (C2) gewonnenen, konzentriertes Monomer bzw. konzentrierte Monomere umfassenden Stroms (5) gefolgt von einem Trennungsschritt zur Gewinnung von nicht kondensierbaren Verbindungen (11) einerseits und eines Stroms von Monomer bzw. Monomeren (8) andererseits umfasst, wobei ein Teil (9) des Stroms von Monomer bzw. Monomeren wieder in den Kopf der Kolonne (C2) eingeleitet wird.

8. Verfahren nach Anspruch 7, **dadurch gekennzeichnet, dass** die nicht kondensierbaren Verbindungen (11) zum Sumpf der Kolonne (C1) zurückgeführt werden.

9. Verfahren nach einem der vorhergehenden Ansprüche, **dadurch gekennzeichnet, dass** der am Sumpf der Kolonne (C1) gewonnene flüssige Strom, der das Extraktionslösungsmittel und Monomer bzw. Monomere umfasst, einem Trennschritt zur Abtrennung eines Teils der nicht kondensierbaren Verbindungen von dem Lösungsmittel unterworfen wird, wobei die nicht kondensierbaren Verbindungen wieder in den Sumpf der Kolonne (C1) eingetragen werden.

10. Verfahren nach einem der Ansprüche 1 bis 8, **dadurch gekennzeichnet, dass** ein Teil des am Sumpf der Kolonne (C1) gewonnenen flüssigen Stroms, der Extraktionslösungsmittel und Monomer bzw. Monomere umfasst, in einem Wärmetauscher E6 verdampft und wieder in den Sumpf der Kolonne (C1) eingetragen wird.

## Claims

1. Process for the recovery of one or more monomers from a gas stream (1), comprising the following stages:
within one and the same first extraction column C1,
a) a stage of extraction by bringing the gas stream (1) into contact, in an extraction column (C1), with an organic extraction solvent (2), in which the said extraction solvent (2) absorbs the said monomer or monomers, and
b) a stage of stripping or desorption with an inert gas in the extraction column (C1), by feeding, at the bottom of the column (C1) and below the feeding of the gas stream (1) laden with monomers, with a stream of inert gas, preferably molecular nitrogen or a gas stream enriched in molecular nitrogen (12),
a liquid stream (3) comprising the extraction solvent and the monomer or monomers being recovered at the bottom of the column (C1) and an exiting gas stream (4) being recovered at the top of the column (C1), then
within a second recovery column C2,
c) a stage of recovery of the said monomer or monomers, in which the said monomer or monomers are separated from the extraction solvent by distillation in a recovery column (C2) fed with the liquid stream (3) recovered at the bottom of the column (C1), a stream comprising concentrated monomer(s) (5) being recovered at the top of the column (C2) and a liquid stream (2) comprising the extraction solvent being recovered at the bottom of the column (C2) and then recycled to the top of the column (C1),
the monomer or monomers being chosen from dienes, vinylaromatic compounds and isobutene.

2. Process according to Claim 1, **characterized in that** the monomer or monomers are chosen from isoprene, butadiene, isobutene and styrene.

3. Process according to Claim 1 or 2, **characterized in that** the extraction solvent is chosen from aliphatic, cycloaliphatic and aromatic hydrocarbons having from 5 to 20 carbon atoms.

4. Process according to Claim 3, **characterized in that** the extraction solvent is chosen from n-hexane, hexane isomer fractions, cyclohexane, n-heptane, heptane isomer fractions, methylcyclohexane and toluene.

5. Process according to any one of the preceding claims, **characterized in that** it comprises a stage of condensation of the exiting gas stream (4) recovered at the top of the column (C1) followed by a separation stage in order to recover, on the one hand, a gas purge (6) and, on the other hand, a solution (7) of condensed hydrocarbons comprising extraction solvent and monomer or monomers, the said solution (7) being recycled at the top of the column (C1).

6. Process according to any one of the preceding claims, **characterized in that** the liquid stream (3) comprising the extraction solvent and monomer or monomers recovered at the bottom of the column (C1) is heated in a heat exchanger E3 by the liquid stream (2) comprising the extraction solvent recovered at the bottom of the column (C2).

7. Process according to any one of the preceding claims, **characterized in that** it comprises a stage of condensation of the stream comprising concentrated monomer(s) (5) recovered at the top of the column (C2), followed by a separation stage in order to recover, on the one hand, incondensable compounds (11) and, on the other hand, a stream of monomer(s) (8), a portion (9) of the stream of monomer(s) being reinjected at the top of the column (C2).

8. Process according to Claim 7, **characterized in that** the incondensable compounds (11) are recycled at the bottom of the column (C1).

9. Process according to any one of the preceding claims, **characterized in that** the liquid stream comprising the extraction solvent and monomer or monomers recovered at the bottom of the column (C1) is subjected to a separation stage in order to separate, from the said solvent, a portion of the incondensable compounds, the said incondensable compounds being reintroduced at the bottom of the column (C1).

10. Process according to any one of Claims 1 to 8, **characterized in that** a portion of the liquid stream comprising the extraction solvent and monomer or monomers recovered at the bottom of the column (C1) is evaporated in a heat exchanger E6 and is reintroduced at the bottom of the column (C1).
